Europäisches Patentamt

European Patent Office

Office européen des brevets

(19)

(11) **EP 0 611 185 B1**

(12) **FASCICULE DE BREVET EUROPEEN**

(45) Date de publication et mention
de la délivrance du brevet:
**12.06.1996 Bulletin 1996/24**

(51) Int. Cl.⁶: **C07C 5/27**, B01J 21/20

(21) Numéro de dépôt: **94400203.9**

(22) Date de dépôt: **31.01.1994**

(54) **Procédé continu pour l'isomérisation des oléfines avec régénération du catalyseur**

Kontinuierliches Verfahren zur Isomerisierung von Olefinen mit Katalysatorregenerierung

Continuous process for the isomerisation of olefins comprising the regeneration of the catalyst

(84) Etats contractants désignés:
**BE DE ES FR GB IT NL**

(30) Priorité: **09.02.1993 FR 9301389**

(43) Date de publication de la demande:
**17.08.1994 Bulletin 1994/33**

(73) Titulaire: **INSTITUT FRANCAIS DU PETROLE**
**F-92502 Rueil-Malmaison (FR)**

(72) Inventeurs:
• **Burzynski, Jean-Pierre**
**F-69110 Sainte-Foy-Les-Lyon (FR)**
• **Duee, Didier**
**F-95610 Eragny Sur Oise (FR)**

• **Debonneville, Jean**
**F-78380 Bougival (FR)**
• **Travers, Christine**
**F-92500 Rueil Malmaison (FR)**
• **Mank, Larry**
**F-78630 Orgeval (FR)**

(74) Mandataire: **Andreeff, François**
**INSTITUT FRANCAIS DU PETROLE**
**4, avenue de Bois-Préau**
**92502 Rueil-Malmaison (FR)**

(56) Documents cités:
EP-A- 0 032 543          FR-A- 2 484 400
US-A- 3 558 733          US-A- 4 225 419

## Description

La présente invention concerne un procédé continu d'isomérisation squelettale avec régénération du catalyseur à base d'alumine et de titane.

## ART ANTERIEUR

La réduction des alkyles de plomb dans les essences a conduit le raffineur à envisager depuis quelques années, l'incorporation à l'essence de différents composés et en particulier, des alcools et des éthers, permettant d'augmenter l'indice d'octane. Outre le méthanol qui est l'un des plus intéressants additifs connu, le MTBE (méthyl-tertio-butyl éther) et le TAME (tertio-amyl-méthyl éther), possèdent des propriétés antidétonantes permettant l'amélioration de la qualité des essences et l'augmentation de leur indice d'octane, augmentation supérieure à celle obtenue avec le méthanol. Ces deux additifs possèdent également de nombreux autres avantages tels que :

- un point d'ébullition correspondant à celui des composants de l'essence qui présentent les plus faibles propriétés antidétonantes,
- une tension de vapeur compatible avec les composants précités,
- un excellent point de congélation,
- une solubilité dans l'eau faible,
- une miscibilité complète avec les hydrocarbures.

Le MTBE est généralement obtenu à partir d'isobutène et de méthanol.
Le TAME est quant à lui obtenu à partir des iso-amylènes et du méthanol.
L'isobutène et les isopentènes utilisés sont généralement contenus dans les coupes oléfiniques $C_3$-$C_4$-$C_5$, issues des effluents du craquage catalytique et du vapocraquage. Cependant les quantités d'isobutène et d'isopentènes fournies par ces différents procédés ne sont pas suffisantes pour permettre un large développement des procédés de production de MTBE et de TAME.

C'est pourquoi, il a été proposé, afin de produire de plus grandes quantités d'isobutène et d'isopentènes, d'isomériser complètement ou presque en isobutène et en isopentènes, les butènes et pentènes contenus dans les effluents des procédés cités ci-dessus.

De nombreux procédés associés à de nombreux catalyseurs ont été proposés qui mettent en oeuvre des catalyseurs à base d'alumine, ou plus particulièrement d'alumines activées ou traitées à la vapeur, d'alumines halogénées, de bauxite ou d'alumine traitées avec des dérivés du bore, du silicium ou du zirconium. Ainsi par exemple le brevet US-3,558,733 décrit un procédé d'isomérisation squelettale avec un catalyseur d'alumine (eta ou gamma) ou bien d'alumine halogénée ou borée, à 315 - 595° C. Lorsque l'activité du catalyseur est réduite à une valeur inacceptable, l'apport de charge oléfinique est interrompu et le catalyseur est régénéré. Il est calciné à environ 600° C (480 à 650° C) en présence d'oxygène, puis il est refroidi à 315 - 480° C avant d'être traité par de la vapeur d'eau. La quantité d'eau ajoutée pour ce traitement est telle que la teneur en eau du catalyseur se situe entre 0,75 et 2,5 %. Puis ce catalyseur est remis en contact de la charge, en présence d'eau (5 - 100 ppm) toujours de façon à maintenir une quantité d'eau de 0,75 à 2,5 % en contact du catalyseur.

Dans un autre brevet EP-32.543, il est décrit un procédé d'isomérisation squelettale avec un catalyseur d'alumine fluorée. Ce catalyseur nécessite après régénération en présence de vapeur d'eau, un traitement supplémentaire avec un composé fluoré, pour maintenir la teneur en fluor sur le catalyseur. La réaction d'isomérisation se déroule avec des quantités énormes d'eau (30 à 400 % molaire).

La plupart de ces procédés présente une conversion par passe relativement faible et une faible sélectivité due aux réactions parasites telles que le craquage et la polymérisation. Il s'ensuit une baisse des performances au cours du temps ce qui entraîne des régénérations fréquentes du catalyseur et donc nécessairement un fonctionnement discontinu des installations, le réacteur ne fonctionnant pas lorsque le catalyseur est en régénération. De plus, les procédés utilisant l'alumine fluorée nécessitent un apport de composé fluoré, ce qui complique le fonctionnement.

## OBJET DE L'INVENTION

La présente invention se propose de remédier à ces inconvénients par l'emploi d'un procédé simple fonctionnant en continu qui permet d'augmenter la production de l'installation et la durée de vie des équipements plus précisément par la réduction des contraintes thermiques et également de diminuer les investissements du fait de la taille réduite des équipements.

Ce procédé utilise de façon originale un catalyseur à base d'alumine et de titane (0,03 à 0,6 %) qui, au contact d'une quantité déterminée de vapeur d'eau (3 - 85 % en poids par rapport au poids de catalyseur), convient particulièrement bien à un procédé continu avec régénération intégrée.

Plus précisément l'invention concerne un procédé d'isomérisation squelettale de n-oléfines contenant au plus 20 atomes de carbone, dans lequel la charge contenant les n-oléfines est, dans une zone de réaction, mise en contact d'un catalyseur à base d'alumine en présence d'eau, et le catalyseur usé est régénéré puis traité à la vapeur d'eau avant d'être remis en contact de la charge, la température dans la zone de réaction étant comprise entre 300 - 600° C, la pression entre 1 - 10 bar, et la vitesse spatiale de la charge entre 0,1 et 10 $h^{-1}$. Ledit procédé continu opère avec circulation du catalyseur essentiellement constitué d'alumine et de 0,03 à 0,6 % de titane (en poids), le catalyseur traversant la zone de réaction à lit mobile étant soutiré pour être régénéré puis traité à la vapeur d'eau à une température sensiblement égale à celle de la zone de réaction, et que la quantité de vapeur d'eau au contact du catalyseur, dans la zone de réaction et pendant le traitement après régénération, s'établit entre 3 et 85 % en poids par rapport au poids du catalyseur, la quantité d'eau introduite dans la zone de réaction étant également dans le rapport molaire eau/charge oléfinique comprise entre 0,1 et 3.

Plus particulièrement la présente invention concerne la production d'iso-oléfines à partir d'une charge comprenant des n-oléfines ayant le plus souvent de 4 à 6 atomes de carbone. Elle concerne plus particulièrement l'isomérisation des n-butènes en isobutènes et des n-pentènes en isopentènes (iso-amylènes).

Le procédé comprend des moyens d'injection de vapeur d'eau après régénération car cela permet de manière surprenante d'améliorer les performances et la stabilité du catalyseur ainsi que sa durée de vie .

La charge circule à travers au moins une zone catalytique, le plus souvent verticale ; cette zone catalytique est le plus souvent constituée d'un lit mobile (ou de plusieurs lits mobiles) à écoulement radial. Elle est par exemple identique à celle décrite dans le brevet US-A-4 277 444. Dans la suite du texte on dénommera zone de réaction l'ensemble des zones de réaction qu'il y en ait une ou plusieurs.

Dans cette zone verticale le catalyseur est en général en mouvement lent descendant et est extrait de manière continue ou bien périodiquement de la dernière zone catalytique pour être envoyé dans une zone de régénération à la sortie de laquelle le catalyseur régénéré est renvoyé en tête de la première zone catalytique afin de maintenir une activité sensiblement constante dans chacune des zones.

On adopte pour le soutirage du catalyseur issu de la dernière zone catalytique une fréquence et un taux convenables. Par exemple le catalyseur peut être soutiré soit en continu, soit périodiquement par exemple, avec une fréquence de 1/10 à 10 jours, en ne soutirant à la fois qu'une fraction par exemple 0,5 à 15 % de la quantité totale de catalyseur. Il est également possible de soutirer ce catalyseur avec une fréquence beaucoup plus rapide de l'ordre de la minute ou de la seconde ; la quantité soutirée est alors réduite en proportion.

D'une façon générale, dans le procédé continu selon l'invention, le catalyseur peut circuler de façon continue entre la zone de réaction, la régénération, le traitement à la vapeur, la zone de réaction à nouveau. La circulation peut également avoir lieu, à certains moments, de manière périodique mais à une fréquence relativement rapide, ainsi que décrit par exemple pour le soutirage du catalyseur de la zone de réaction.

La circulation de catalyseur est adaptée de façon à maintenir une activité quasi constante du catalyseur dans le réacteur.

A cet effet, le dispositif peut comporter des moyens pour le transfert périodique du catalyseur entre la zone de réaction et le réacteur de régénération et/ou entre le réacteur de régénération et la zone de traitement à la vapeur et/ou entre le réacteur de régénération et la zone de réaction et/ou entre la zone de traitement à la vapeur et la zone de réaction.

Le catalyseur usé ou partiellement usé, provenant de la dernière zone catalytique est alors dirigé par gravité vers un ballon "accumulateur" situé au dessous de la dernière zone catalytique et au dessus de la zone de régénération.

La régénération du catalyseur peut être effectuée en mode discontinu ou en mode continu par tout moyen connu. En mode discontinu le catalyseur usé ou partiellement usé s'accumule dans le ballon "accumulateur" avant d'alimenter le régénérateur situé généralement en dessous de ce ballon. A intervalles réguliers le régénérateur est mis en équilibre de pression par exemple sous atmosphère de vapeur d'eau ou d'azote avec le ballon "accumulateur décanteur" il est ensuite rempli de catalyseur provenant à travers un système de vannes du ballon "accumulateur" puis isolé du reste du système. Le régénérateur est alors purgé à l'azote ou à la vapeur d'eau pour éliminer les hydrocarbures et l'on effectue la régénération du catalyseur. Le régénérateur, ou un ballon accumulateur dans lequel on a transféré le catalyseur régénéré, est ensuite purgé à l'azote ou à la vapeur d'eau, puis mis sous vapeur d'eau en équilibre de pression avec la zone catalytique dans laquelle le catalyseur va être injecté.

La régénération proprement dite du catalyseur est effectuée par combustion des dépôts de coke. Cette opération est réalisée par l'injection d'un mélange contenant de l'air dilué par de la vapeur d'eau ou de l'azote. La teneur en oxygène dans le gaz de régénération injecté en tête du régénérateur est de préférence comprise entre 0.01 et 2 % en volume. L'oxygène du mélange de gaz injecté est consommé par la combustion des dépôts de coke et la fin de la combustion se détecte aisément par l'augmentation de la teneur en oxygène dans le gaz sortant du régénérateur et également par la disparition du front de flamme (plan horizontal où se produit la combustion) qui se propage de haut en bas du lit catalytique. La combustion est réalisée à une température moyenne comprise de préférence entre 350 et 550 °C et sous une pression comprise, par exemple, entre 1 et 15 bars. La durée de ce traitement peut être comprise entre 20 minutes et trois heures. En général, elle est d'environ 1 heure. Il est particulièrement indiqué dans le procédé selon l'invention, de régénérer à une température sensiblement égale ($\pm$ 40° C et encore mieux $\pm$ 20° C) à celle régnant dans la zone de réaction.

Le catalyseur est ensuite soumis à un traitement à la vapeur d'eau habituellement à une température comprise entre 110 °C et 700 °C et de manière préférée entre 200 et 600 °C et de manière encore plus avantageuse à une température sensiblement égale (± 40° C et encore mieux ± 20° C) à celle régnant dans la zone de réaction, sous une pression partielle de vapeur d'eau supérieure à 0,1 bar et de préférence comprise entre 0,6 et 4 bars pendant une durée comprise entre 6 minutes et 10 heures et de préférence de 30 minutes à trois heures.

Ce traitement permet de maximiser la proportion des sites de Bronsted sur le catalyseur alumine/titane lorsque la quantité de vapeur d'eau en contact du catalyseur s'établit entre 3 et 85 % en poids (par rapport au poids de catalyseur), et de préférence 10 - 60 %, et encore plus avantageusement 10 - 40 % ou 18 - 35 %.

Ces traitements s'effectuent soit dans une enceinte à lit fixe de catalyseur, soit dans une enceinte à lit mobile.

Le catalyseur régénéré est alors remonté par un élévateur à gaz ("lift") vers un ballon "accumulateur décanteur" situé au dessus du réacteur. Le fluide de l'élévateur utilisé pour convoyer le catalyseur peut être n'importe quel fluide inerte et très avantageusement de la vapeur d'eau.

Dans le ballon "accumulateur décanteur" la vapeur d'eau est séparé du catalyseur et dirigée vers un filtre pour éliminer les particules fines de catalyseur, et de là envoyée vers un compresseur en vue de son recyclage vers l'espace de réaction, vers les élévateurs ou la zone de régénération.

Selon une variante du procédé la zone de traitement à la vapeur d'eau peut être placée après le ballon accumulateur décanteur et avant la première zone catalytique

Le catalyseur frais et/ou régénéré alimente progressivement, au fur et à mesure du soutirage du catalyseur usé, la zone de réaction choisie ou la zone de traitement à la vapeur, afin de maintenir une activité quasi constante dans le réacteur.

La charge à isomériser est mise au contact du catalyseur à une température comprise entre 300 °C et 600 °C (et de préférence entre 400 et 550 °C lorsqu'elle est constituée de butènes et/ou de pentènes) à une pression comprise entre 1 et 10 bars absolus (et préférentiellement entre 1 et 5 bars absolus lorsque la charge est constituée de n-butènes et/ou de n-pentènes).

La vitesse spatiale est comprise entre 0,1 et 10 $h^{-1}$ exprimée en volume de charge oléfinique par volume de catalyseur et par heure (et de manière préférée entre 0,5 et 6 $h^{-1}$ lorsque la charge est constituée de butènes et/ou de pentènes).

Selon l'invention, le procédé est mis en oeuvre en présence d'eau, afin de minimiser les réactions secondaires indésirables. La quantité d'eau introduite dans le réacteur est telle que le rapport molaire $H_2O$/charge oléfinique soit compris entre 0,1 et 3 (et préférentiellement entre 0,3 et 2 et encore plus avantageusement entre 0,5 et 2, par exemple lorsque la charge est constituée de n-butènes et/ou de n-pentènes).

Cet apport d'eau permet de maintenir la quantité de vapeur d'eau en contact du catalyseur à 10 - 40 % en poids (par rapport au poids de catalyseur) pour conserver les performances du catalyseur.

Le catalyseur employé dans la présente invention est un catalyseur constitué essentiellement (en-dehors de l'eau éventuellement fixée) d'alumine et de 0,03 à 0,6 % en poids de titane. Le catalyseur est de préférence sous forme de grains.

Les grains du catalyseur ont le plus souvent la forme de billes sensiblement sphériques de diamètre compris généralement entre 1 et 3 millimètres (mm) et de préférence entre 1,5 et 2 mm. La surface spécifique du support est le plus souvent compris entre 10 et 500 $m^2$/g et de préférence entre 50 et 450 $m^2$/g, son volume poreux étant compris entre 0,4 et 0,8 $cm^3$/g.

Le catalyseur neuf est préférentiellement traité avant toute utilisation sous vapeur d'eau avant d'être mis au contact des hydrocarbures, ledit traitement ayant lieu entre 110 et 700 °C sous une pression partielle de vapeur d'eau supérieure à 0,1 bar pendant une durée de 0,1 à 120 heures.

L'invention concerne également un dispositif pour l'isomérisation de n-oléfines contenant au plus 20 atomes de carbone, ledit dispositif comportant :

- au moins un réacteur d'isomérisation (1) contenant un catalyseur à base d'alumine en lit mobile, muni d'une conduite (15) pour l'introduction de catalyseur, d'une conduite (2) pour son extraction, d'une conduite (16) pour l'introduction de la charge contenant les n-oléfines et d'une conduite (17) pour l'extraction de la charge traitée,
- un régénérateur (6) muni d'une conduite (5) pour l'introduction du catalyseur usé, et d'une conduite (7) pour l'extraction du catalyseur régénéré, de moyens pour l'introduction du gaz de régénération et pour l'évacuation des gaz formés,
- un élévateur pneumatique (10, 11) avec une conduite (9) d'amenée du gaz moteur, et arrivant dans un ballon accumulateur décanteur (12).

De façon avantageuse, le régénérateur est muni d'une conduite pour l'introduction de vapeur d'eau.

Entre le réacteur d'isomérisation et le ballon accumulateur décanteur (12), on dispose avantageusement un réacteur (14) pour le traitement à la vapeur d'eau du catalyseur régénéré introduit par la conduite (13), ledit réacteur comportant une conduite (15) pour le transfert du catalyseur traité vers le réacteur d'isomérisation.

Ce dispositif peut comporter des moyens pour le transfert périodique du catalyseur entre le réacteur (1) et le régénérateur (6). Il comporte également soit des moyens pour le transfert périodique du catalyseur entre le régénérateur (6) et le réacteur (1) soit des moyens pour le transfert périodique du catalyseur entre les réacteurs (14) et (1).

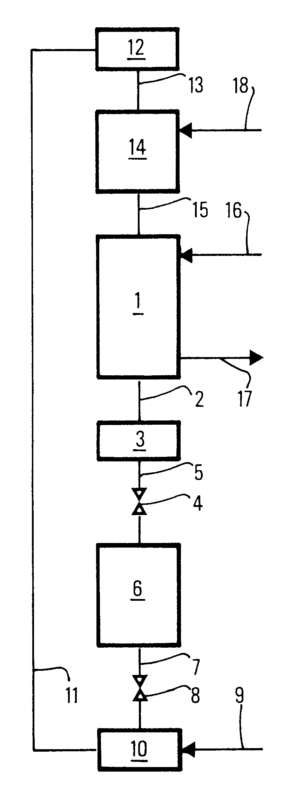

La figure 1 illustre schématiquement l'invention sans toutefois en limiter la portée. Dans la réalisation schématisée sur la figure 1 une seule zone de réaction a été représentée où le catalyseur circule de haut en bas.

La charge entre en haut du réacteur (1) et en sort par le bas. Le catalyseur neuf est introduit par le conduit (15) en haut du réacteur (1), et le catalyseur usé est soutiré par la conduite (2) pour être dirigé à travers le ballon accumulateur (3) et la ligne (5) vers la zone de régénération (6). Le régénérateur (6) est mis en équilibre de pression avec le réacteur sous atmosphère de vapeur d'eau ou d'azote. Le régénérateur est alors isolé du reste du système par fermeture des vannes (4) et (8) et la combustion du coke décrite ci-avant y est conduite en lit fixe ou bien en lit mobile.

Dans une première variante du procédé cette étape est suivie du traitement à la vapeur décrit plus haut. Lorsque ce traitement est terminé, le régénérateur est mis sous équilibre de pression avec le réacteur; le catalyseur est dirigé par la ligne (7) vers un ballon tampon (10), remonté par un élévateur pneumatique (11), fonctionnant avec un gaz moteur introduit par la ligne (9) qui est préférentiellement de la vapeur d'eau vers le ballon accumulateur décanteur (12). Le catalyseur régénéré et prétraité à la vapeur d'eau est introduit directement par la ligne (13,15) dans le réacteur (1).

Dans une seconde variante du procédé seule l'étape de combustion du coke est réalisé dans le régénérateur (6). Le catalyseur débarrassé du coke est dirigé par la ligne (7) vers le ballon tampon (10), remonté par un élévateur (11), fonctionnant avec le gaz moteur introduit par la ligne (9) qui est de la vapeur d'eau ou de l'azote vers le ballon accumulateur décanteur (12) puis dirigé par la ligne (13) vers une zone (ou réacteur) (14) où il est traité à la vapeur dans les conditions décrits ci-avant. Le catalyseur régénéré et prétraité à la vapeur d'eau est introduit progressivement dans le réacteur (1) par la ligne (15).

Le procédé continu selon la présente invention permet des gains de productivité importants, une durée de vie des équipements prolongée et également des coûts d'investissement diminués du fait de la taille réduite des équipements, par rapport à un procédé discontinu.

Ce dernier avantage est tout à fait significatif au niveau du traitement à la vapeur qui nécessite ici des quantités de vapeur moindres du fait des quantités inférieures régénérées par passe.

L'invention est particulièrement avantageuse lorsqu'un catalyseur alumine/titane est utilisé, le taux de cokage de ce catalyseur étant réduit l'utilisation de la boucle de régénération en continu permet un fonctionnement des réacteurs à un niveau d'activité élevé et un maintien au cours du temps de ce niveau et activité.

Les exemples qui suivent précisent l'invention sans toutefois en limiter la portée.

Les performances en isomérisation seront exprimées par

1 / la conversion des butènes

$$C = \frac{\Sigma(\% \text{ n-butènes}) \text{ charge} - \Sigma (\% \text{ n-butènes}) \text{ effluent}}{\Sigma(\% \text{ n-butènes}) \text{ charge}} \times 100$$

2 / la sélectivité en isobutènes

$$\Sigma = \frac{(\% \text{ isobutène}) \text{ effluent} - (\% \text{ isobutène}) \text{ charge}}{\Sigma (\% \text{ n-butènes}) \text{ charge} - \Sigma (\% \text{ n-butènes effluent})} \times 100$$

Le catalyseur utilisé dans ces exemples est un support d'alumine gamma commercial imprégné de 0,1 % de titane à partir d'oxalate de titane décahydraté en solution aqueuse puis séché à 100 °C pendant 2 heures et calciné à 600 °C pendant 2 heures.

Le catalyseur est préalablement soumis à un traitement à la vapeur d'eau à 560 °C pendant 20 heures avec une pression partielle de vapeur d'eau égale à 0,8 bar.

La charge d'hydrocarbure que l'on traite a la composition précisée ci-après dans le tableau 1

Tableau 1

| Charge | % poids |
|--------|---------|
| $iC_4$ | 0,216 |
| $nC_4$ | 21,76 |
| $C_4$=2TR | 2,732 |
| $C_4$=1 | 63,878 |
| $iC_4$= | 5,617 |
| $C_4$=2Cis | 5,139 |
| $C_4$== | 0,03 |
| $C_5$+ | 0,628 |

On a opéré avec un réacteur du type "radial" tel que décrit dans le brevet au nom de la demanderesse US-A-4 277 444; la température d'entrée est de 530 °C et la vitesse volumique spatiale est de 2 volume de charge liquide (à 15 °C) par volume de catalyseur et par heure. Le taux de vapeur d'eau injecté est de 2 mole par mole de coupe $C_4$ qui représente environ 0,6 g/g de catalyseur. La pression à l'entrée du réacteur est de 1,8 bar.

La quantité de catalyseur est de 55 m³. Le catalyseur est renouvelé à un débit de 380 kg/h. Le débit de vapeur dans le lift est de 10 kg/h.

EXEMPLE 1 (comparatif)

Dans cet exemple le catalyseur usé issu du réacteur (1) est soumis à une régénération par combustion du coke dans les conditions suivantes :

la température à l'entrée de la zone de régénération (6) est maintenue à 490 °C, la pression dans le régénérateur (6) à 2 bars, la teneur en oxygène du mélange gazeux (azote plus air) introduit est maintenue à 0,5 % en volume et le temps de séjour dans cette zone est de 1h30.

Le régénérateur est ensuite purgé à l'azote puis mis sous atmosphère d'azote en équilibre de pression avec le réacteur.

Le catalyseur se désactive rapidement au cours des cycles ce qui se traduit par une chute spectaculaire de la conversion et par voie de conséquence une chute impressionnante du rendement en isobutène. Les résultats obtenus sont précisés dans le tableau 2 ci-après.

Tableau 2

| Heures de marche (h) | Conversion $nC_4$= (%poids) | Sélectivité $iC_4$= (%poids) | Rendement $iC_4$= (%poids) |
|----------------------|-----------------------------|------------------------------|----------------------------|
| 250 | 31,2 | 83,5 | 26,0 |
| 1250 | 27,5 | 85,3 | 23,5 |
| 1350 | 26,5 | 86,8 | 23,0 |

EXEMPLE 2 (selon l'invention)

Un lot de catalyseur de même formulation est utilisé.

Le même dispositif et les mêmes conditions que celles décrites en liaison avec l'exemple 1 sont utilisés.

Le protocole de régénération est identique excepté la réalisation d'une phase de traitement à la vapeur d'eau dans le régénérateur (6) après la combustion du coke.

Celle-ci est effectuée à 530 °C sous une pression de vapeur d'eau de 2 bars pendant une durée de 1 heure.

La quantité de vapeur d'eau en contact du catalyseur est de 0,6 g/g de catalyseur.

Le catalyseur est ensuite transporté par l'élévateur ("lift") (11) sous atmosphère de vapeur d'eau dans le réacteur (1).

Dans ces conditions le catalyseur se révèle très stable au cours des cycles, ce qui se traduit par un maintien de la conversion à un niveau élevé, une sélectivité en isobutène constante au cours des cycles et par voie de conséquence un rendement en isobutène sensiblement constant au cours du temps. Les résultats obtenus sont précisés dans le tableau 3 ci-après.

Tableau 3

| Heures de marche (h) | Conversion $nC_4$= (%poids) | Sélectivité $iC_4$= (%poids) | Rendement $iC_4$= (%poids) |
|---|---|---|---|
| 250 | 33,7 | 83,1 | 28,0 |
| 1250 | 33,5 | 83,3 | 27,9 |
| 1350 | 33,6 | 82,8 | 27,8 |

Dans les différents tableaux, $C_2$ désigne l'éthane, $C_2$= : l'éthylène, $C_3$ : le propane, $iC_4$ : l'isobutane, $nC_4$ : le n-butane, $C_4$=2TR : le butène-2trans, $C_4$=1 : le butène-1, $iC_4$= : l'isobutène, $C_4$=2Cis : le butène-2cis, $C_4$== : le butadiène et $C_5$+ : les hydrocarbures à 5 atomes de carbone et plus.

EXEMPLE 3 (Comparatif avec US-3,558,733)

L'exemple 3 diffère de l'exemple 2 en ce que le catalyseur utilisé est une alumine y commerciale seule, c'est-à-dire non imprégnée de Titane. Ce catalyseur est soumis, avant tout contact avec la charge, à un traitement à la vapeur d'eau à 560° C pendant 20 heures avec une pression partielle de vapeur d'eau faible de 0,04 bar.

La charge d'hydrocarbures a la composition précisée dans le tableau (1). Les conditions opératoires sont les mêmes que celles utilisées dans l'exemple 2, si ce n'est que le taux de vapeur injecté est très faible et égal à 0,2 mole par mole de coupe $C_4$. La quantité de vapeur d'eau au contact du catalyseur est alors de 0,06 g/g de catalyseur.

Toutes les autres opérations sont identiques à celles décrites dans l'exemple 2. Les performances sont données Tableau 4. La stabilité et la sélectivité du catalyseur se révèlent bien plus faibles que celles obtenues avec un catalyseur alumine/titane mis en oeuvre avec un rapport molaire $H_2O$/coupe $C_4$= élevé.

Tableau 4

| Heures de marche (h) | Conversion $nC_4$= (%poids) | Sélectivité $iC_4$= (%poids) | Rendement $iC_4$= (%poids) |
|---|---|---|---|
| 250 | 44,2 | 60,5 | 26,75 |
| 1250 | 40,0 | 64,3 | 25,7 |
| 1350 | 37,5 | 66,2 | 24,8 |

En conclusion, l'exemple 1 montre également clairement la nécessité du traitement à la vapeur après la régénération, avec une quantité d'eau suffisante et à une température proche de celle de la zone de réaction.

L'exemple comparatif 3 montre clairement que avec un catalyseur de l'art antérieur (US-3,588,733) à base d'alumine ne contenant pas de titane, régénéré par calcination suivi de traitement à la vapeur mais avec des quantités d'eau différentes, les performances obtenues en isomérisation squelettale sont bien au-dessous de celles obtenues par le procédé selon l'invention.

Outre ces performances supérieures, le procédé présente également l'avantage d'être simple dans sa mise en oeuvre (pas d'apport de fluor, comme dans le cas des catalyseurs fluorés) et fiable au niveau de l'appareillage (contraintes thermiques minimisées du fait que la température reste sensiblement constante dans toutes les étapes du procédé).

**Revendications**

1. Procédé d'isomérisation squelettale de n-oléfines contenant au plus 20 atomes de carbone, dans lequel la charge contenant les n-oléfines est, dans une zone de réaction, mise en contact d'un catalyseur à base d'alumine en présence d'eau, et le catalyseur usé est régénéré puis traité à la vapeur d'eau avant d'être remis en contact de la charge, la température dans la zone de réaction étant comprise entre 300 - 600° C, la pression entre 1 - 10 bar, et la vitesse spatiale de la charge entre 0,1 et 10 $h^{-1}$, caractérisé en ce que le procédé continu opère avec circulation du catalyseur essentiellement constitué d'alumine et de 0,03 à 0,6 % de titane (en poids), le catalyseur traversant la zone de réaction à lit mobile étant soutiré pour être régénéré puis traité à la vapeur d'eau à une température sensiblement égale à celle de la zone de réaction, et que la quantité de vapeur d'eau au contact du catalyseur, dans la zone de réaction et pendant le traitement après régénération, s'établit entre 3 et 85 % en poids par rapport au poids du catalyseur, la quantité d'eau introduite dans la zone de réaction étant également dans le rapport molaire eau/charge oléfinique compris entre 0,1 et 3.

2. Procédé selon la revendication 1, caractérisé en ce que le catalyseur circule sous atmosphère de vapeur d'eau.

3. Procédé selon l'une des revendications précédentes, caractérisé en ce que le traitement à la vapeur d'eau est effectué entre 110 et 700 °C, sous une pression partielle de vapeur d'eau supérieure à 0,1 bar et pendant une durée de 6 mn à 10 heures.

4. Procédé selon l'une de revendications précédentes, caractérisé en ce que la régénération du catalyseur est effectuée par injection d'un gaz contenant de l'oxygène, à une température de 350-550 °C et sous une pression comprise entre 1 et 15 bars.

5. Procédé selon l'une des revendications précédentes, caractérisé en ce que la zone de réaction est constituée de lit(s) mobile(s) à écoulement radial.

6. Procédé selon l'une des revendications précédentes, caractérisé en ce que le catalyseur est extrait périodiquement de la zone de réaction.

7. Procédé selon d'une des revendications précédentes, caractérisé en ce que la régénération est effectuée en mode discontinu.

8. Procédé selon l'une des revendications précédentes, caractérisé en ce que le traitement à la vapeur est effectué en mode discontinu.

9. Procédé selon l'une des revendications précédentes, caractérisé en ce que la circulation du catalyseur est continue.

10. Dispositif pour l'isomérisation de n-oléfines contenant au plus 20 atomes de carbones, ledit dispositif comportant

- au moins un réacteur d'isomérisation (1) contenant un catalyseur à base d'alumine en lit mobile, muni d'une conduite (15) pour l'introduction de catalyseur, d'une conduite (2) pour son extraction, d'une conduite (16) pour l'introduction de la charge contenant les n-oléfines et d'une conduite (17) pour l'extraction de la charge traitée,
- un régénérateur (6) muni d'une conduite (5) pour l'introduction du catalyseur usé, et d'une conduite (7) pour l'extraction du catalyseur régénéré, de moyens pour l'introduction du gaz de régénération et pour l'évacuation des gaz formés,
- un élévateur pneumatique (10,11) avec une conduite (9) d'amenée du gaz moteur, et arrivant dans un ballon accumulateur décanteur (12).

11. Dispositif selon la revendication 10, dans lequel le régénérateur est muni d'une conduite pour l'introduction de vapeur d'eau.

12. Dispositif selon la revendication 10, comportant, entre le réacteur d'isomérisation et le ballon accumulateur décanteur (12), un réacteur (14) pour le traitement à la vapeur d'eau du catalyseur régénéré introduit par la conduite (13), ledit réacteur comportant une conduite (18) pour l'introduction d'eau et une conduite (15) pour le transfert du catalyseur traité vers le réacteur d'isomérisation.

13. Dispositif selon l'une des revendications précédentes, comportant des moyens pour le transfert périodique du catalyseur entre le réacteur (1) et le régénérateur (6).

14. Dispositif selon l'une des revendications précédentes, comportant des moyens pour le transfert périodique du catalyseur entre le régénérateur (6) et le réacteur (1).

15. Dispositif selon l'une des revendications 10 à 13, comportant des moyens pour le transfert périodique du catalyseur entre les réacteurs (14) et (1).

**Claims**

1.   Process for the skeletal isomerization of n-olefins containing at the most 20 carbon atoms, in which the charge containing the n-olefins is, in a reaction zone, contacted with an alumina-based catalyst in the presence of water and the spent catalyst is regenerated and then treated with steam before being recontacted with the charge, the temperature in the reaction zone being between 300 and 600°C, the pressure between 1 and 10 bars, the space velocity of the charge between 0.1 and 10 h$^{-1}$, characterized in that the continuous process takes place with a flow of the catalyst essentially constituted by alumina and 0.03 to 0.6 wt. % of titanium, the catalyst passing through the moving bed reaction zone being drawn off for regeneration and then treatment with steam at a temperature substantially equal to that of the reaction zone and the steam quantity in contact with the catalyst, in the reaction zone and during the treatment after regeneration is at between 3 and 85 wt.% based on the catalyst weight, the quantity of water introduced into the reaction zone also being in a water/olefin charge molar ratio between 0.1 and 3.

2.   Process according to claim 1, characterized in that the catalyst flows under a steam atmosphere.

3.   Process according to either of the preceding claims, characterized in that the steam treatment is performed at between 110 and 700°C under a partial steam pressure above 0.1 bar and for between 6 min and 10 hours.

4.   Process according to any one of the preceding claims, characterized in that the catalyst is regenerated by injecting a gas containing oxygen, at a temperature of 350 to 550°C and under a pressure between 1 and 15 bars.

5.   Process according to any one of the preceding claims, characterized in that the reaction zone is constituted by radial flow moving bed or beds.

6.   Process according to any one of the preceding claims, characterized in that the catalyst is periodically extracted from the reaction zone.

7.   Process according to any one of the preceding claims, characterized in that the regeneration is performed batchwise.

8.   Process according to any one of the preceding claims, characterized in that the steam treatment is performed batchwise.

9.   Process according to any one of the preceding claims, characterized in that the catalyst flow is continuous.

10.  Apparatus for the isomerization of n-olefins containing at the most 20 carbon atoms, said apparatus comprising at least one isomerization reactor (1) containing an alumina-based catalyst in moving bed, provided with a pipe (15) for the introduction of the catalyst, a pipe (2) for its extraction, a pipe (16) for the introduction of the charge containing the n-olefins and a pipe (17) for the extraction of the treated charge; a regenerator (6) equipped with a pipe (5) for the introduction of the spent catalyst and a pipe (7) for the extraction of the regenerated catalyst, means for the introduction of the regeneration gas and for the discharge of the gases formed; a pneumatic lift (10, 11) with a motor gas supply pipe (9) and passing into a storage/settling balloon flask (12).

11.  Apparatus according to claim 10, wherein the regenerator is provided with a steam introduction pipe.

12.  Apparatus according to claim 10 comprising, between the isomerization reactor and the storage/settling balloon flask (12), a reactor (14) for the steam treatment of the regenerated catalyst introduced by the pipe (13), said reactor having a water introduction pipe (18) and a pipe (15) for the transfer of the treated catalyst to the isomerization reactor.

13.  Apparatus according to any one of the preceding claims comprising means for the periodic transfer of the catalyst between the reactor (1) and the regenerator (6).

14.  Apparatus according to any one of the preceding claims comprising means for the periodic transfer of the catalyst between the regenerator (6) and the reactor (1).

15.  Apparatus according to any one of the claims 10 to 13 comprising means for the periodic transfer of the catalyst between the reactors (14) and (1).

**Patentansprüche**

1. Verfahren zur Skelettisomerisierung von n-Olefinen, welche höchstens 20 Kohlenstoffatome enthalten, worin die die n-Olefine enthaltende Charge in einer Reaktionszone mit einem Katalysator auf Basis von Aluminiumoxid in Gegenwart von Wasser in Kontakt gebracht wird und der verbrauchte Katalysator regeneriert, dann mit dem Wasserdampf behandelt wird, bevor er wieder in Kontakt mit der Charge gebracht wird, wobei die Temperatur in der Reaktionszone zwischen einschließlich 300 bis 600°C, der Druck zwischen einschließlich 1 bis 10 bar und die Raumgeschwindigkeit der Charge zwischen einschließlich 0,1 und 10 $h^{-1}$ enthalten ist, dadurch gekennzeichnet, daß das kontinuierliche Verfahren unter Zirkulation des im wesentlichen aus Aluminiumoxid und von 0,03 bis 0,6 % Titan (in Gewicht) bestehenden Katalysators arbeitet, wobei der die Reaktionszone mit Fließbett durchlaufende Katalysator zum Regenerieren, dann Behandeln mit dem Wasserdampf bei einer Temperatur, die im wesentlichen gleich jener der Reaktionszone ist, abgezogen wird, und daß sich die Menge an Wasserdampf im Kontakt mit dem Katalysator in der Reaktionszone und während der Behandlung nach der Regenerierung auf zwischen 3 bis 85 Gew.%, bezogen auf das Gewicht des Katalysators, einstellt, wobei die in die Reaktionszone eingeführte Wassermenge sich ebenfalls im Molverhältnis Wasser/olefinische Charge, das zwischen einschließlich 0,1 und 3 enthalten ist, befindet.

2. Verfahren nach Anspruch 1, dadurch gekennzeichnet, daß der Katalysator unter Wasserdampfatmosphäre zirkuliert.

3. Verfahren nach einem der vorhergehenden Ansprüche, dadurch gekennzeichnet, daß die Behandlung mit Wasserdampf zwischen einschließlich 110 und 700°C, unter einem Partialdruck von Wasserdampf von über 0,1 bar, während einer Zeitdauer von 6 Minuten bis 10 Stunden stattfindet.

4. Verfahren nach einem der vorhergehenden Ansprüche, dadurch gekennzeichnet, daß die Regenerierung des Katalysators durch Injektion eines Gases, das Sauerstoff enthält, bei einer Temperatur von 350 bis 550°C und unter einem Druck, der zwischen einschließlich 1 und 15 bar enthalten ist, stattfindet.

5. Verfahren nach einem der vorhergehenden Ansprüche, dadurch gekennzeichnet, daß die Reaktionszone aus einem oder mehreren Fließbetten mit radialer Strömung bestehen.

6. Verfahren nach einem der vorhergehenden Ansprüche, dadurch gekennzeichnet, daß der Katalysator periodisch aus der Reaktionszone abgezogen wird.

7. Verfahren nach einem der vorhergehenden Ansprüche, dadurch gekennzeichnet, daß die Regenerierung in diskontinuierlicher Weise bewirkt wird.

8. Verfahren nach einem der vorhergehenden Ansprüche, dadurch gekennzeichnet, daß die Behandlung mit Dampf in diskontinuierlicher Weise bewirkt wird.

9. Verfahren nach einem der vorhergehenden Ansprüche, dadurch gekennzeichnet, daß das Zirkulieren des Katalysators kontinuierlich ist.

10. Vorrichtung zur Isomerisierung von n-Olefinen, welche höchstens 20 Kohlenstoffatome enthalten, wobei die Vorrichtung

   - wenigstens einen Isomerisierungsreaktor (1), der einen Katalysator auf Basis von Aluminiumoxid im Fließbett enthält, der mit einer Leitung (15) zum Einführen des Katalysators, einer Leitung (2) zu seinem Abziehen, einer Leitung (16) zum Einführen der die n-Olefine enthaltenden Charge und einer Leitung (17) zum Abziehen der behandelten Charge ausgerüstet ist,

   - einen Regenerator (6), ausgerüstet mit einer Leitung (5) zum Einführen des verbrauchten Katalysators und einer Leitung (7) zum Abziehen des regenerierten Katalysators, Mittel zum Einführen von Regenerierungsgas und zum Evakuieren von gebildeten Gasen,

   - einen pneumatischen Elevator (10, 11) mit einer Leitung (9) zum Zuführen von Antriebsgas, welcher in einem Sammeldekanteur-Ballon (12) endet, umfaßt.

**11.** Vorrichtung nach Anspruch 10, worin dar Regenerator mit einer Leitung zum Zuführen von Wasserdampf ausgerüstet ist.

**12.** Vorrichtung nach Anspruch 10, welche mischen dem Isomerisierungsreaktor und dem Sammeldekanteur-Ballon (12) einen Reaktor (14) zur Behandlung des durch die Leitung (13) eingeführten regenerierten Katalysator mit Wasserdampf umfaßt, wobei der Reaktor eine Leitung (18) zum Einführen von Wasser und eine Leitung (15) für die Überleitung des behandelten Katalysators in den Isomerisierungsreaktor umfaßt.

**13.** Vorrichtung nach einem der vorhergehenden Ansprüche, welche Mittel zur periodischen Überleitung des Katalysators zwischen dem Reaktor (1) und dem Regenerator (6) umfaßt.

**14.** Vorrichtung nach einem der vorhergehenden Ansprüche, welche Mittel für die periodische Überleitung des Katalysators zwischen dem Regenerator (6) und dem Reaktor (1) umfaßt.

**15.** Vorrichtung nach einem der Ansprüche 10 bis 13, welche Mittel zur periodischen Überleitung des Katalysators zwischen den Reaktoren (14) und (1) umfaßt.